# EUROPEAN PATENT SPECIFICATION

(11) **EP 3 328 344 B1**
(45) Date of publication and mention of the grant of the patent: **27.03.2019**
(21) Application number: 15747780.3
(22) Date of filing: 30.07.2015
(51) Int. Cl.: A61K 8/02, A61Q 1/00, A61Q 19/00

(54) **IMPREGNATED ARTICLE WITH FIBERS AND SCREEN NET**
IMPREGNIERTER KOSMETISCHER ARTIKEL MIT FASERN UND SCHUTZNETZ
MATERIEL COSMETIQUE BASEE SUR UN SUBSTRAT DES FIBRES NON TISSÉS ET UNE RESEAU

(43) Date of publication of application: 06.06.2018
(73) Proprietor: L'Oréal, 75008 Paris (FR)
(72) Inventor: LECHANOINE, Marc, Kawasaki Kanagawa 213-0012 (JP)
(74) Representative: Bourdeau, Françoise
(86) International application number: PCT/EP2015/067542
(87) International publication number: WO 2017/016608

(56) References cited:
- EP-A1- 1 634 566
- EP-A1- 2 837 306
- WO-A1-99/13860
- WO-A2-2004/112734

## Description

The present invention relates to an impregnated cosmetic article comprising a screen net and a fiber carrier impregnated with a cosmetic composition and to a method for producing the same.

The expression "cosmetic product" is understood to mean a product as defined in Council Directive 93/35/EEC of 14 June 1993.

In the entire description, the expression "*comprising a*" should be considered as being synonymous with "*comprising at least one*", unless specified to the contrary.

More particularly, the invention relates an impregnated cosmetic article for applying a cosmetic composition, for example a care product or a makeup product such as a foundation or a cream.

A liquid foundation system in a compact format called "CUSHION foundation" first appeared in Korea in 2008 and is beginning to successfully spread across the world market. This system comprises a foam carrier (sponge) as a carrier which is impregnated with a liquid foundation and enclosed in a container. However, this system could be improved.

Such systems are frequently used by first applying the liquid foundation to fingertips of the user or to a secondary applicator (i.e., cotton ball, cotton swab, pad, etc.), and then to the face.

WO2009/148048 discloses a skin covering sheet for application in a face mask. This skin covering sheet is composed of a non-woven fabric and is impregnated with a cosmetic liquid. The nonwoven fabric is obtained by mixing composite fibers comprising at least a polyethylene component and another polymer component. The mixture is then blended with hydrophilic fibers and hydro-entangled.

JP 2000-287746 discloses a cosmetic puff comprising a pair of urethane sponge layer and a layer of nonwoven fabric bonded to respective surfaces of the sponge layers. However, such a puff leaves traces of the product on the fingers and/or on the secondary applicator after every application, which may be both messy and involve a waste of makeup product in some situations.

WO2004/112734 discloses a cosmetic cleansing pad.

Impregnated cosmetic articles comprising a screen net and an absorber impregnated with a cosmetic composition are known in the art.

EP0528705 discloses an impregnated cosmetic article comprising a spongy element impregnated with a fluid cosmetic product. The spongy element is preferably constituted by flexible synthetic foam with open cells. It can be made of a polyurethane, polyvinyl chloride, polyethylene, epoxy resin or polystyrene. Preferably, the spongy element is surmounted by a flexible screen net. This screen net is held on the spongy element by a frame compressing the periphery of said spongy element. The screen net is made of any material sufficiently flexible to be able to deform the spongy element when pressed thereon and then retrieve to its original shape when the user stops pressing.

JP-U-H01-131422 discloses a member in which a fiber piece is infused with a liquid. A nonwoven plate and synthetic resin sheet are laminated sequentially. The nonwoven plate can have a plate-like shape. The synthetic resin sheet has a surface shape, which is generally the same as the nonwoven plate, and is formed by soft synthetic resins such as polyethylene. Many pores can be drilled in the synthetic resin sheet.

EP 2 837 306 discloses an impregnated cosmetic article comprising a screen net and an absorbing material impregnated with a cosmetic composition. The absorbing material is adjacent to the screen net. The screen net is made of one or more materials selected from the group consisting of polyurethane, TPE (thermoplastic elastomer), polyester, polyether, acryl, and olefin. The absorbing material can be for example a sponge, foam or a non-woven.

There exists a need to further improve articles comprising a screen net and an absorber impregnated with a cosmetic composition.

In particular, there is a need to provide such articles that respond to at least one of the following objective:
- increase comfort during application of a liquid or jelly composition,
- allow a composition to be applied in a precise and, if desired, homogenous way on the skin of the user,
- reduce leakages of the composition out of the absorber,
- allow getting a controlled amount of composition each time the consumer presses on the article,
- keep nearly constant with the time the composition's grade of UV protection,
- keep nearly constant with the time the composition's quantity of preservatives,
- Decrease the rate of absorption of a cosmetic composition on the absorbent,
- increase the rates of restitution of the composition impregnated on the absorbent,
- increase the elasticity of the article after a representative number of uses of the article, particularly after 300 uses of the article
- optimize and simplify the filling process.

The present invention aims to meet at least one of these needs and relates to an impregnated cosmetic article comprising:
(i) a screen net,
(ii) a fiber carrier impregnated with a cosmetic composition,
the article having:
- a thickness in a range between 1 mm and 200 mm,
- a surface density between 30 g/m² and 5500 g/m²,
- an elasticity between 50 % and 100 %.

According to the invention, the "*thickness*" and the "*surface density*" of the article is measured before impregnation of the composition.

"*Elasticity*" of the article is measured after impregnation of the composition and before the first use.

The special choice of the parameters of the article leads to a significant increase of the quality of the article compared to these of prior art. The user benefits from an article that is more comfortable to use and that allows a more precise makeup or care of the body or the face. The leakage is reduced to avoid a waste of the composition. The SPF (sun protection factor) of the composition is overall maintained at its original level during the use of the article. The quantity of preservatives is also kept quite constant with the time.

In particular, the inventors have demonstrated that by choosing the parameters of the article in the specific range indicated previously, one can observe significant improvements. These improvements are relatives at least to one of the following properties:
- Absorption rate,
- Restitution rate,
- Elasticity after 300 uses.

The shape of the fiber carrier is not limited, but is preferably be in the form of a plate or a block such as a cylinder.

In yet another of its aspect, the present invention relates to a cosmetic product comprising an impregnated cosmetic article as described above and a container for said article. Preferably, the cosmetic product further comprises a cosmetic tool to take up the cosmetic composition. More preferable, it comprises a mat positioned under the impregnated cosmetic article.

Preferably, the viscosity of the composition may be between about 1,000 and 20,000 cps. That is, the range of the viscosity is very broad, and thus various types of compositions can be used.

In a first embodiment according to the invention, the viscosity of the composition is between about 1 000 and 10 000 cps. The cosmetic composition is considered to have a low viscosity.

In a second embodiment according to the invention, the viscosity of the composition is between about 10 000 and 20 000 cps. The cosmetic composition is considered to have a high viscosity.

Preferably, the screen net is designed to form a pick-up surface of the cosmetic composition impregnated in the fiber carrier as it passes through holes of the screen net.

The screen net structure is capable to copy the deformation of the fiber carrier when the user presses the impregnated article.

According to the invention, an ejection amount of the cosmetic composition can be controlled by ejecting the cosmetic composition through the holes of the screen net.

The impregnated article according to the invention allows permanent control of the ejection of the cosmetic composition thanks to the arrangement of screen net and fiber carrier. The impregnated article according to the invention enables optimal use of a cosmetic composition impregnated in a fiber carrier. It provides a totally innovative feeling for the consumer. This feeling even remains with the time after several uses of the impregnated article.

### Main definitions

Hereafter, all the tests, testing and measurements are carried out at a temperature of 20°C and at a pressure of 10⁵ Pa and at a humidity rate of 50 %.

Hereafter, the "testing" is done by applying a constant load of 0.4 kgf for 1 sec on an article. The constant load is homogeneous. It is applied on the screen net and covers the whole screen net.

A "*fiber carrier*" is an absorbing substrate comprising fibers.

The "*hardness*" of the article is measured with a durometer hardness tester (type F; manufactured by ASKER). Preferably, the hardness of the article is in the range of 13 to 70 as measured with a durometer hardness tester, type F.

The measurement of the "*viscosity*" is performed using a Contraves TV or Rheomat 180 viscometer equipped with a spindle rotating at 200 t / min. The skilled person can select the spindle for measuring the viscosity from mobile, M1 or M2 on the basis of his general knowledge, so as to carry out the measurement. The measurement is achieved after 10 minutes rotation of the spindle at 200 revolutions / minute in the composition.

The "*surface density*" is defined as the mass of the article before impregnation of the composition divided by the exterior surface of the screen net. The exterior surface of the screen net is perpendicular to the longitudinal axis of the article. The exterior surface of the screen net comes in contact wholly or partially with the fingers or with a cosmetic tool.

The derived SI unit of the surface density measurement is kilogram per square meter (kg/m²).

The term "*restitution rate*" here represents the quantity of cosmetic composition that can be given back by the article when the user presses it.

The restitution rate is: R=(Wo-Wi)/Wf×100; where "Wo" is the article weight before testing, "Wi" is the article weight after testing, and "Wf' is the weight of the cosmetic composition impregnated into the article.

The restitution rate is measured before the first use of the article.

The term "*absorption rate*" represents the quantity of cosmetic composition that can be absorbed by the article.

Absorption rate: A = [(Wsat/Vsat]; where "Wsat" is the weight of the composition that is necessary to saturate a non-impregnated article having a volume Vsat.

The "*elasticity*" is calculated with the following ratio:
E = [(ti/t0] * 100 where t0 is the article thickness before the testing and ti is the elasticity thickness after the testing.

"*Elasticity*@*t0*" is the elasticity of the impregnated cosmetic article before the first use.

"*Elasticity* @*tn*" is the elasticity of the impregnated cosmetic article after 300 uses.

According to the invention, the fiber carrier can be a nonwoven. But this is not complusary.

By "*nonwoven*", it is meant, in the sense of this invention, a substrate including fibers in which the individual fibers or filaments are arranged in a disordered manner in a sheet-like structure and which are neither woven nor knit. The fibers of the nonwoven body are generally linked to one another, under the effect of a mechanical action (for example, by needle-punching, air jet, water jet, etc.), or under the effect of a thermal action, or by adding a binder.

Such a nonwoven material is, for example, defined by standard ISO 9092, as a web or a sheet of fibers oriented directionally or at random, bound by friction and/or cohesion and/or adhesion, excluding paper or products obtained by weaving, knitting, tufting or stitching incorporating threads or bonding filaments.

The nonwoven body is produced based on fibers. Advantageously, the mass percentage of hot-melt fibers contained in the nonwoven sheet is greater than 0.5% and is less than or equal to 100%, advantageously between 15% and 80%.

The hot-melt fibers are, for example, polyolefin fibers, such as polyethylene (PE), polypropylene (PP), polyethylene terephthalate (PET) or acrylic fibers, such as polymethyl methacrylate (PMMA), polyurethane fibers or the following thermoplastic fibers: polyvinyl chloride (PVC), styrene polymers (for example polystyrene PS, expandable polystyrene EPS, acrylonitrile butadiene styrene ABS terpolymer, styrene acrylonitrile copolymer, styrene butadiene SB polymer), polyamides (PA), polycarbonates (PC), saturated polyesters (for example, polyethylene terephthalate glycol PET, polybutylene terephthalate glycol PBT), polyacetals (for example, polyoxymethylene POM, trioxane ethylene oxide copolymer), polyvinyl alcohol (PVA), or fluorinated polymers (for example polytetrafluoroethylene PTFE, polyvinylidene fluoride PVDF, and polychlorotrifluoroethylene PCTFE) fibers.

A "*screen net*" is a material having openings and used for sifting out the cosmetic composition impregnated on the fiber carrier. The openings can be distributed randomly or regularly on the screen net. The screen net directs the flow of the cosmetic composition from the fiber carrier to the exterior of the article. The screen net can be for example a porous material or a sieve. The structure of the screen net can be of any kind.

### Method for manufacturing the impregnated cosmetic article

The invention also relates to a method for manufacturing an impregnated cosmetic article comprising the step of:
(i) Producing layers of fiber carriers,
(ii) Cutting the layers of fiber carriers,
(iii) Placing a screen net on the layers of fiber carriers,
(iv) Adhering the screen net to the fiber carriers,
(v) Impregnating a cosmetic composition.

### 1) Method for manufacturing the non-impregnated cosmetic article

A fiber carrier is generally produced in wide layers and cut at the right thickness, in order to form a sheet with the desired thickness. Generally, the surface of the sheet is 1 m².

Then a screen net can be placed on top of the surface of the fiber carrier, in order to cover it all.

The screen net can be then melted or glued or assembled with an iron plate covering its whole surface, in order to adhere it the fiber carrier.

It can be finally cut to reach a final shape that can fit with a container.

### 2) Method for impregnating the cosmetic composition

The cosmetic composition can be filled in a container.

The non-impregnated cosmetic article can be put on top of the surface of the cosmetic composition.

The top surface of the non-impregnated cosmetic article can be pressed slowly.

A ring can be plugged to the container in order to maintain the impregnated article in the container.

### Detailed description of preferred embodiments

The invention can be better understood from the following detailed description of non-limiting embodiments thereof, and on examining the accompanying drawings, in which:
Fig. 1 is a disassembled oblique view of a cosmetic product according to one of the embodiments of the present invention;
Fig. 2 is a disassembled oblique view of a cosmetic product according to another of the embodiments of the present invention;

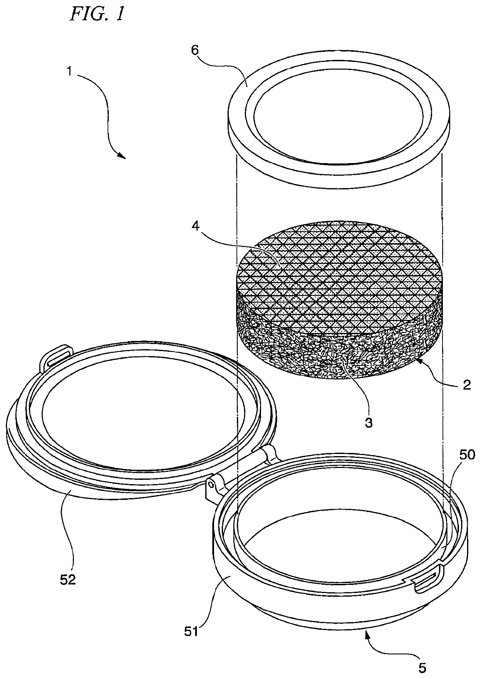

The cosmetic product 1 shown in Figure 1 is a liquid foundation system in a compact format. The cosmetic product 1 includes an impregnated article 2 comprising a fiber carrier 3 and a screen net 4 covering an exterior surface (upper surface) of the fiber carrier 3, a container 5 for the article 2, and a sealing member 6. The fiber carrier 3 is impregnated with a cosmetic fluid composition, such as a liquid, creamy, or gel composition. The term "fluid" here means that the cosmetic composition is capable of flowing and changing its shape at a steady rate when acted upon by a force to change its shape, preferably is a liquid, at a temperature of from 20 to 40°C, and preferably an ambient temperature, i.e., 25°C.

The container 5 comprises a storage part 50 for enclosing the impregnated article 2, a peripheral wall part 51 surrounding the storage part 50, and a lid part 52 which can be put on the storage part 50 and the peripheral wall part 51, and which is hingedly connected with the peripheral wall part 51.

The sealing member 6 has a ring shape, which is fitted on an upper edge of the storage part 50 and exposes the impregnated article 2. The sealing member 6 is capable of preventing the cosmetic fluid composition from leaking from the upper edge of the storage part 50 and pressing a periphery of the screen net 4 to retain the article 2 within the storage part 50. The sealing member 6 can be pressed but it is not compulsory. It is just better under certain conditions such as formula rheology.

The impregnated article 2 has a shape conforming to an internal shape of the storage part 50. The screen net 4 is fabric, net, film with openings/orifices/pores/porosity, or the like which has permeability for the cosmetic composition across the whole sheet. The screen net 4 is attached to the upper surface of the fiber carrier 3 such that it is integral with the fiber carrier 3. Alternatively, the fiber carrier 3 and the screen net 4 may be constituted separately. In addition, an additional cover sheet, such as a metallic net, may be positioned on the screen net 4.

The fiber carrier 3 is a fiber block composed of a set of fibers organized randomly or non-randomly. The fiber carrier 3 is porous, and can absorb a cosmetic fluid composition. The fiber carrier 3 may comprise a network structure of pores. The size of the pores is not limited, and may be from 0.1 to 5 mm, preferably from 0.3 to 4 mm, and more preferably from 0.6 to 3 mm. The composition according to the present invention can be stored in the pores.

The length and width of the fibers are not limited. The length may be from 0.5 to 20 mm, preferably from 1 to 15 mm, and more preferably from 3 to 10 mm. The width of the fibers may be from 1 to 500 µm, preferably from 10 to 400 µm, and more preferably from 50 to 300 µm.

The material of the fibers is not limited. Preferably, the fiber material comprises a non woven. The fibers may be prepared from synthetic resins such as polyolefins (in particular, polyethylene and polypropylene), polyesters, and polyethylene terephthalate; or natural fibers such as cotton, cellulose and silk. Preferably, the fiber carrier 3 is made with polyethylene fibers, polypropylene fibers, polyethylene terephthalate fibers, or a mixture thereof. Herewith, even if the cosmetic fluid composition includes UV filters or preservatives, neither the sunscreen SPF level nor the effects of the preservatives will deteriorate since these fibers are capable of no or low absorption of UV filters or preservatives. Preferably, the cosmetic composition comprises a UV absorbing agent or a preservative.

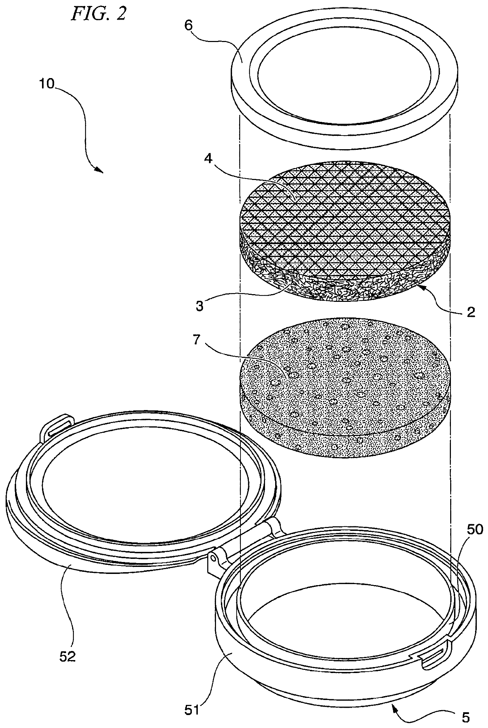

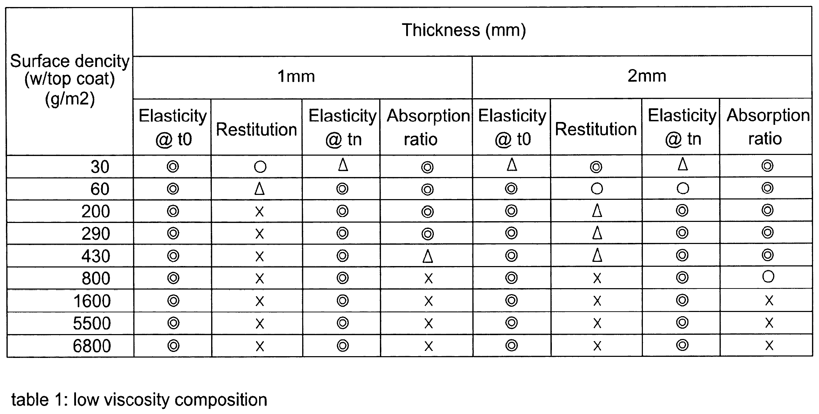

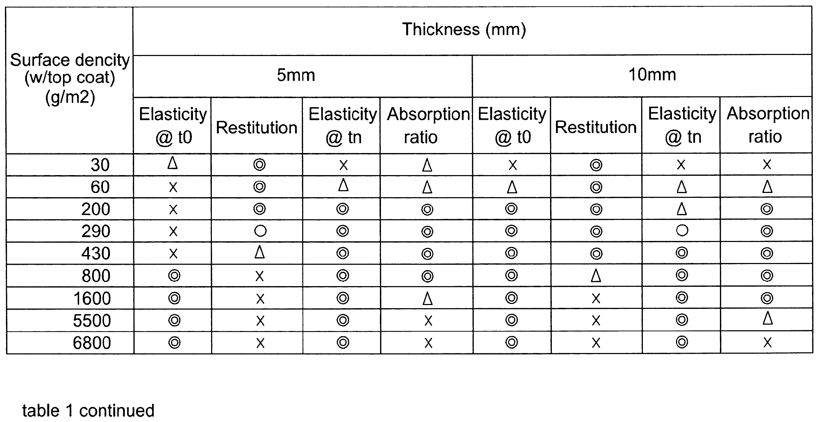

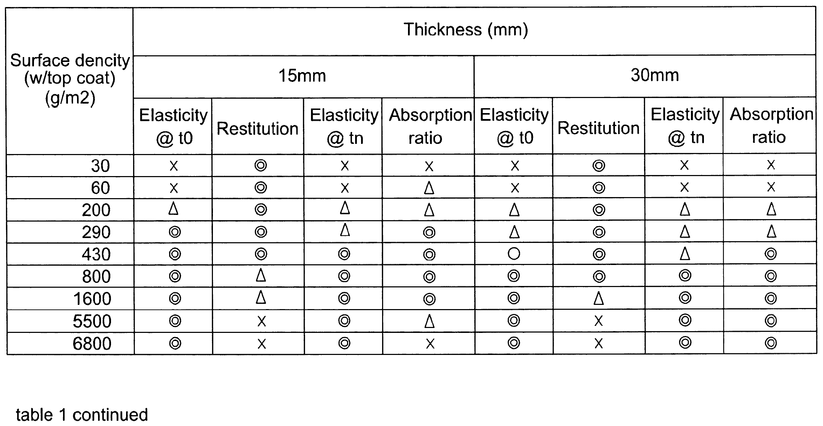

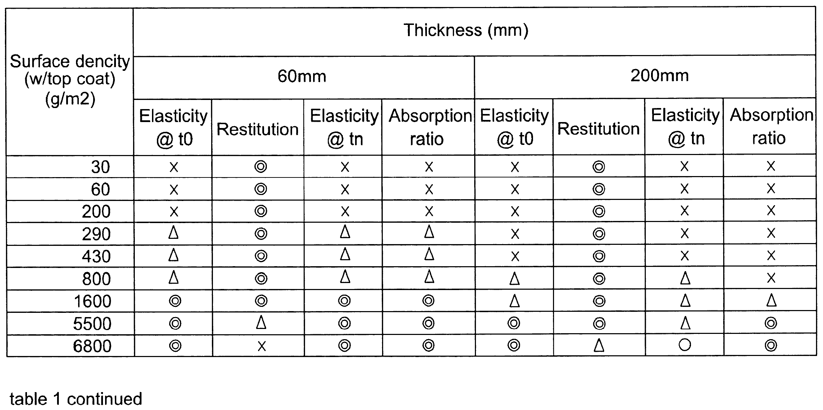

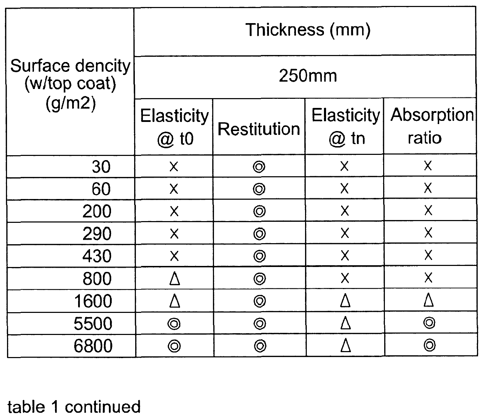

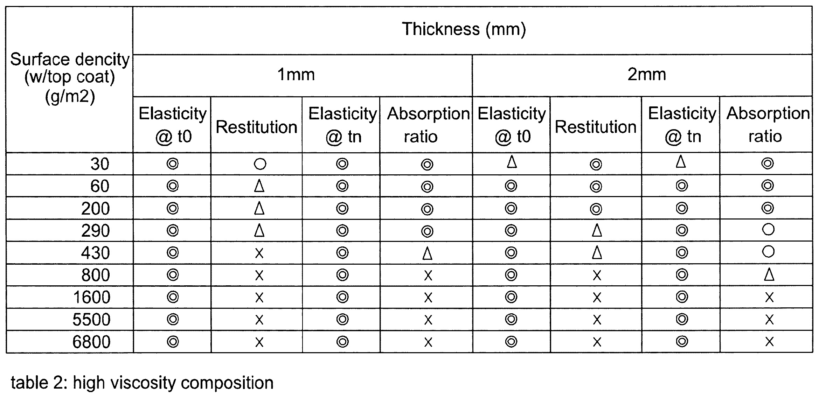

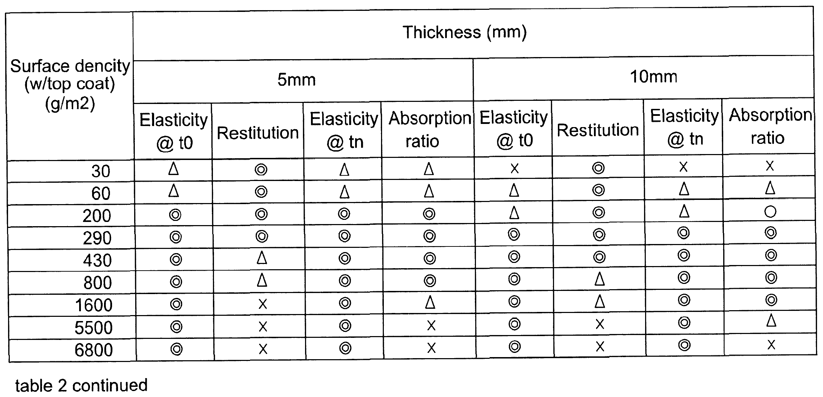

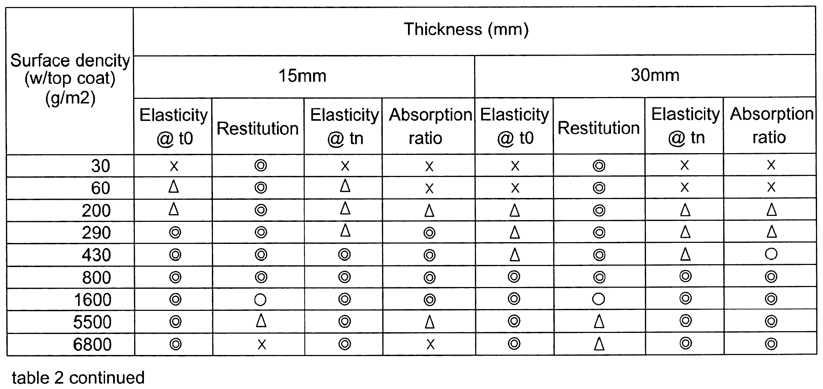

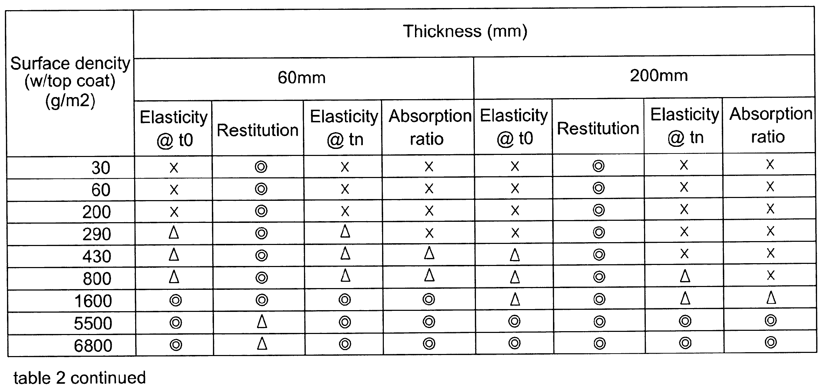

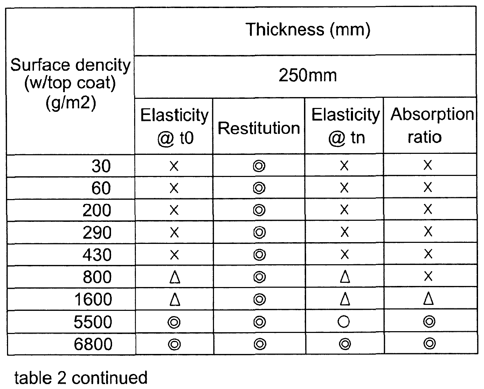

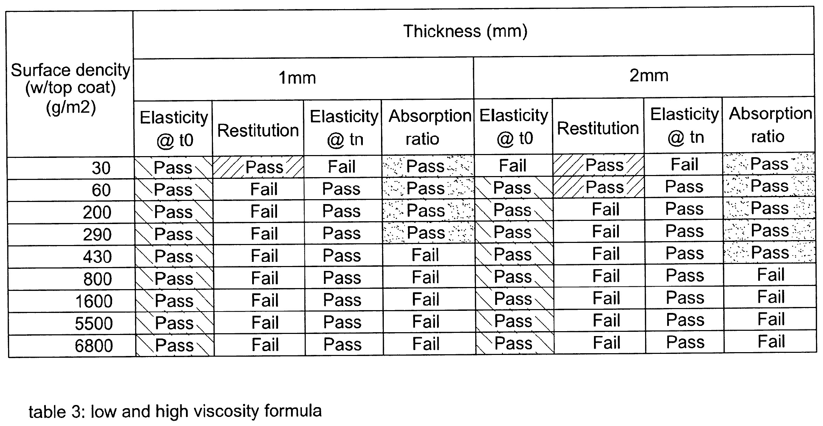

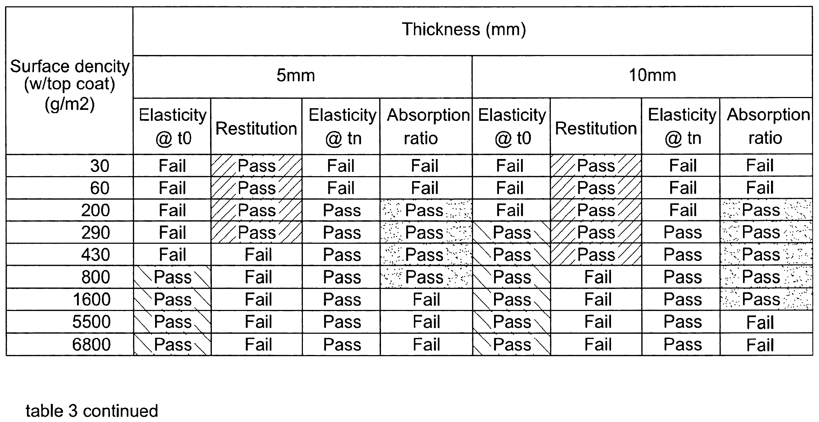

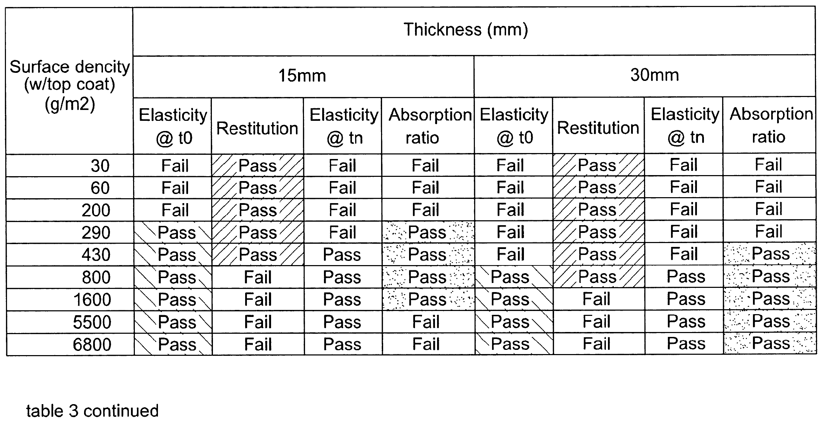

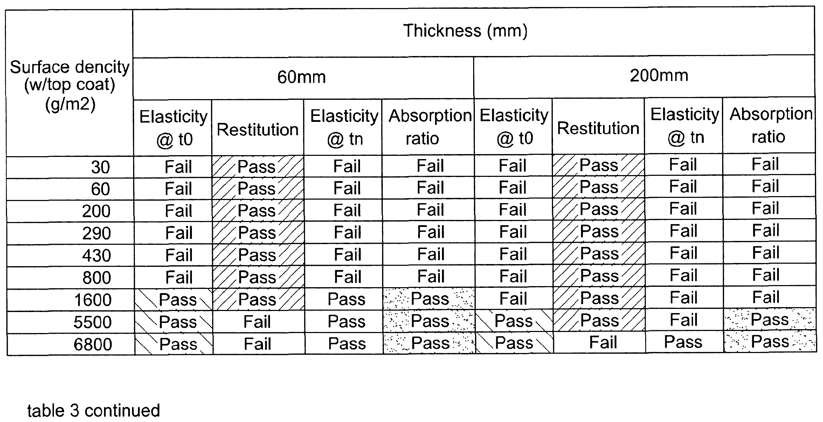

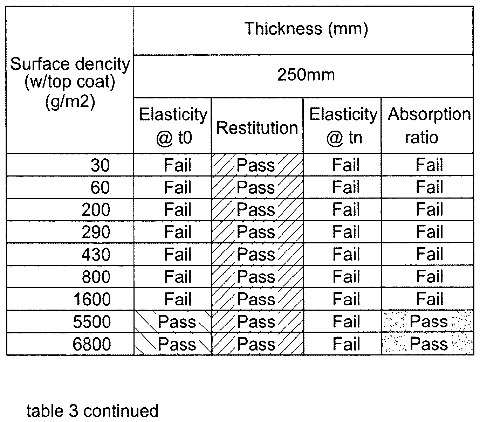

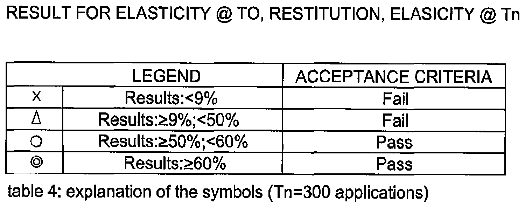

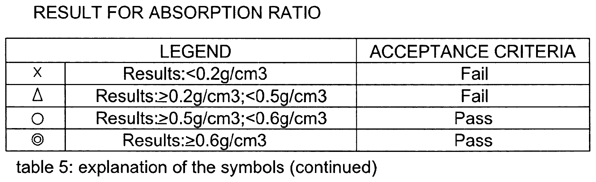

The fibers can be of different natures and can be mixed at the same time to maximize the permeability of the fiber carrier 3, its restitution (use up ratio) and its elasticity, which will enable compatibility with the cosmetic fluid composition having a wide range of viscosities. The fiber carrier 3 shown in Figures 1 and 2 has a circular shape, but is not limited thereto.

A user of the cosmetic product 1 can take the cosmetic fluid composition from the fiber carrier 3 with a tool such as a puff. For example, by applying the tool onto the fiber carrier 3, with or without pushing the tool to the fiber carrier 3, the composition in the fiber carrier 3 can be transferred to the tool from the carrier. The user may take the cosmetic fluid composition from the fiber carrier 3 with his/her hand.

Figure 2 shows another embodiment of the cosmetic product according to the invention. The cosmetic product 10 shown in Figure 2 is also a liquid foundation system in a compact format. The cosmetic product 10 further comprises a honeycomb mat 7 with springiness in addition to the above-mentioned components, that is, the impregnated article 2, the container 5, the sealing member 6, and the cosmetic fluid composition. The honeycomb mat 7 is a sponge made of polyurethane, etc. The honeycomb mat 7 is enclosed in the storage part 50 and is positioned under the article 2. The honeycomb mat 7 includes many cells communicating with each other, and is also impregnated with the cosmetic fluid composition. The honeycomb mat 7 directly contacts with the fiber carrier 3, and is in fluid communication with the fiber carrier.

Preferably, the screen net 4 comprises a polyolefin, a polyester, a polyurethane, an acryl or an epoxy resin. More preferably, the screen net comprises a polyolefin. Even more preferably, the fiber carrier 3 adheres to the screen net 4.

The screen net 4 may be connected to the fiber carrier 3 by thermic treatment and calendaring.

The thickness of the screen net 4 is preferably within the range between 0,01 mm and 0,5 mm, and even more preferably within the range between 0,05 mm and 0,3mm.

The surface of each of the hole of the screen net may be preferably from 0,05 mm² to 0,50 mm². It may different according to the rheology of the cosmetic composition. The repartition of the holes over the surface can be organized or randomized, till the proportion of holes preferably remains at least over 30 %.

The thickness of the fiber carrier 3 is preferably within the range between 10 mm and 60 mm, and even more preferably within the range between 13 mm and 30 mm.

The surface density of the fiber carrier 3 is preferably within the range between 200 g/m² and 2000 gm², and even more preferably within the range between 200 g/m² and 1600 g/m².

The restitution rate for the composition impregnated in the fiber carrier 3 is preferably within the range between 40 % and 99 %, and more preferably within the range between 60 % and 99 %.

Preferably, the fiber carrier 3 comprises air cavities and the volume percentage of the air cavities in the fiber carrier is in the range of 35 % to 90 %.

The elasticity of the article 2 is preferably within the range between 40 % and 99 %, and more preferably within the range between 60 % and 99 %.

The absorption rate of the article 2 is preferably within the range between 40 % and 99 %, and more preferably within the range between 60 % and 99 %.

The Asker hardness F of the fiber carrier 3 is preferably 65 ± 13, that is, within the range between 52 and 78. This allows a "cushion" feeling for users when the users touch or press the exterior surface of the fiber carrier 3 either directly or using any tool. In addition, this allows proper bulk flow though the fiber carrier, and therefore, a suitable amount of the cosmetic fluid composition can be transferred through the fiber carrier from the fiber carrier 3 to the tool or skin of the users. The term "bulk flow" here means a flow of the cosmetic fluid composition through pores in the fiber carrier 3. The diameter of fibers is preferably within the range between 1 µm to 100 µm, preferably between 10 µm and 53 µm. This allows the proper ratio of cavities per fiber carrier volume, for optimized impregnability, bouncy effect, and hardness. The percentage of cavities in the fiber carrier 3 is preferably between 35% and 90%, more preferably between 45% and 80%, and even more preferably between 55% and 70%. This allows high impregnability with different types of liquid/creamy/gel compositions.

### Evaluation of impregnated articles

Several impregnated articles with differing thicknesses, surface densities, and elasticity before the first use were prepared.

Impregnated compositions were:
- a low viscosity composition,
- a high viscosity composition.

The elasticity before use, the elasticity after 300 uses, the restitution rate and the absorption rate were evaluated for each articles.

### Description of the article before impregnation

### Fiber carrier

The fiber carrier is made of PP inside and PE outside.

The volume percentage of air cavities in the fiber carrier is 77%.

### Screen net

The screen net is made of polyolefin covered with a hot melt layer of copolymer ethylene-vinyl acetate (EVA).

Its thickness is 0, 12 mm with a size of holes of 0,189 mm².

### Process for the preparation of the non-impregnated article

The fiber carrier is first produced and cut at the right thickness, in order to form a sheet of 1 m² with a thickness of 15 mm.

Then the screen net is placed on top of the surface in order to cover it all.

The screen net is then melted with an iron plate covering the whole surface, in order to melt the EVA hot melt layer and the PP/PE fibers.

It is finally cut to reach the final shape that fits with the container.

### Process for impregnating the cosmetic composition

The cosmetic composition is filled in a container.

The non-impregnated cosmetic article is put on top of the surface of the cosmetic composition.

The screen net of the non-impregnated cosmetic article is pressed slowly.

A ring is plugged to the container in order to maintain the impregnated article in the container.

### Cosmetic compositions

The cosmetic compositions were:
- Low viscosity composition: Shu Uemura TM Cleansing Oil ® (61 ± 5 UD (M1)) called Shu Uemura skin Purifier blanc chroma brightening and polishing gentle cleansing oil ®.
- High viscosity composition: Lancôme Blanc Expert Cushion ® High Coverage SPF 50 (30-40 UD (M1)).

The results of the tests are shown in tables 1 to 3 for various surface density and thickness of articles.

In these tables, the term "*top coat*" means "exterior surface of the screen net".

The conditions for the evaluation of the articles are shown in tables 4 and 5.

The results "pass" or "fail" of the article with respect to the following results:
- the restitution rate,
- the absorption rate,
- the elasticity after 300 applications
depends on three parameters:
- the thickness of the fiber carrier,
- the elasticity before the first use of the article,
- the surface density of the article.

It can be seen that when these three parameters are in the range claimed in the present patent application, either the absorption rate or the restitution rate or the elasticity at after 300 applications passes. This demonstrates the importance of choosing the parameters specifically as found by the inventor to reach the aim of the invention.

In some cases, when the three parameters are in the range claimed in the present patent application, then even two results are reached.

For some cases, even the three results are reached.

Furthermore, regarding potential absorption of the ingredients (UV filters and preservative) in the cosmetic fluid composition, a test carried out by the inventor is explained below.

Two kinds of samples made of different materials (PU and PP/PE) were prepared. The test was performed by contacting each of these samples with the cosmetic fluid composition for a predetermined time, and the UV absorbances of the samples as well as the contents of preservative (phenoxyethanol) in the samples were calculated.

The results of the test were as shown below.

| | UV Absorbance | |
|---|---|---|
| | Deviation from Reference | |
| | Formula Reference: 5.93 | |
| Contact Time | PU | PP/PE |
| 3h | 5.61 (-5.4%) | 5.93 (0%) |
| 24h | 5.44 (-8.3%) | 5.73 (-3.4%) |
| 72h | 5.41 (-8.8%) | 5.57 (-6.1%) |

| | Content of Phenoxyethanol | |
|---|---|---|
| | Deviation from Reference | |
| | Formula Reference: 0.62% | |
| Contact Time | PU | PP/PE |
| 3h | 0.44 (-29%) | 0.61 (-2%) |
| 24h | 0.45 (-27%) | 0.62 (0%) |

As shown in the above results of the test, the reductions in the UV absorbance and the content of phenoxyethanol were confirmed in the PU sample. Contrastingly, neither significant reductions in the UV absorbance nor the content of phenoxyethanol were confirmed in the PP/PE sample.

This system can be used in many variations: Compact cases, jars or even bottles, and it is adaptable to all cosmetic formulas, except for solid formulations. This article can in fact also be used with almost all kinds of cosmetic compositions including make-up, skincare, haircare, hair dye, etc. It can also be integrated into caps or into direct application systems. It is also adaptable to a mask version.

## Claims

1. Impregnated cosmetic article (2) comprising:
- a screen net (4),
- a fiber carrier (3) impregnated with a cosmetic composition, the
article having:
- a thickness in a range between 1 mm and 200 mm,
- a surface density between 30 g/m² and 5500 g/m²,
- an elasticity between 50 % and 100 %
and the screen net (4) being designed to form a pick-up surface of the cosmetic composition impregnated in the fiber carrier (3) as it passes through holes of the screen net (4).

2. Impregnated cosmetic article (2) according to claim 1, wherein the fiber carrier (3) comprises a non-woven material.

3. Impregnated cosmetic article (2) according to any of the preceding claims, wherein the fiber carrier (3) is made with polyethylene fibers, polypropylene fibers, polyethylene terephthalate fibers, or a mixture thereof

4. Impregnated cosmetic article (2) according to any of the preceding claims, wherein the fibers have a diameter between 1 µm to 100 µm.

5. Impregnated cosmetic article (2) according to any of the preceding claims, wherein the fiber carrier (3) comprises air cavities and the volume percentage of the air cavities in the fiber carrier is in the range of 35 % to 90 %.

6. Impregnated cosmetic article (2) according to any of the preceding claims, wherein the screen net (4) comprises a polyolefin.

7. Impregnated cosmetic article (2) according to any of the preceding claims, wherein the fiber carrier (3) adheres to the screen net (4).

8. Impregnated cosmetic article (2) according to any of the preceding claims, the screen net (4) comprising holes having each a surface from 0,05 mm² to 0,50 mm².

9. Impregnated cosmetic article (2) according to any of the preceding claims, having hardness in the range of 13 to 70 as measured with a durometer hardness tester, type F.

10. Impregnated cosmetic article (2) according to any of the preceding claims, wherein the cosmetic composition comprises a UV absorbing agent or a preservative.

11. Impregnated cosmetic article (2) according to any of the preceding claims, wherein the viscosity of the cosmetic composition is between 1,000 and 20,000 cps.

12. A method for manufacturing an impregnated cosmetic article (2) according to any of the preceding claims comprising the step of:
(i) Producing layers of fiber carriers (3),
(ii) Cutting the layers of fiber carriers (3),
(iii) Placing a screen net (4) on the layers of fiber carriers (3),
(iv) Adhering the screen net (4) to the fiber carriers (3),
(v) Impregnating a cosmetic composition.

13. A cosmetic product (10) comprising:
- an impregnated cosmetic article (2) according to any of claims 1 to 11,
- a container (5) for the article (2).

14. A cosmetic product (10) according to claim 13 further comprising a cosmetic tool to take up the cosmetic composition.

15. A cosmetic product according to claim 13 or 14 further comprising a mat (7) positioned under the impregnated cosmetic article (2).

## Patentansprüche

1. Imprägnierter kosmetischer Gegenstand (2), umfassend:
- ein Siebnetz (4),
- einen Faserträger (3), der mit einer kosmetischen Zusammensetzung imprägniert ist,
wobei der Gegenstand aufweist:
- eine Dicke in dem Bereich zwischen 1 mm und 200 mm,
- eine Oberflächendichte zwischen 30 g/m² und 5500 g/m²,
- eine Elastizität zwischen 50 % und 100 %,
und das Siebnetz (4) dafür gestaltet ist, eine Abgabefläche für die in dem Faserträger (3) imprägnierte kosmetischen Zusammensetzung zu bilden, wenn sie durch Löcher des Siebnetzes (4) tritt.

2. Imprägnierter kosmetischer Gegenstand (2) gemäß Anspruch 1, wobei der Faserträger (3) ein nichtgewebtes Material umfasst.

3. Imprägnierter kosmetischer Gegenstand (2) gemäß einem der vorstehenden Ansprüche, wobei der Faserträger (3) mit Polyethylenfasern, Polypropylenfasern, Polyethylenterephthalatfasern oder einem Gemisch davon hergestellt ist.

4. Imprägnierter kosmetischer Gegenstand (2) gemäß einem der vorstehenden Ansprüche, wobei die Fasern einen Durchmesser zwischen 1 µm und 100 µm aufweisen.

5. Imprägnierter kosmetischer Gegenstand (2) gemäß einem der vorstehenden Ansprüche, wobei der Faserträger (3) Lufthohlräume umfasst und der Volumenanteil der Lufthohlräume in dem Faserträger in dem Bereich von 35 % bis 90 % liegt.

6. Imprägnierter kosmetischer Gegenstand (2) gemäß einem der vorstehenden Ansprüche, wobei das Siebnetz (4) ein Polyolefin umfasst.

7. Imprägnierter Kosmetischer Gegenstand (2) gemäß einem der vorstehenden Ansprüche, wobei der Faserträger (3) an dem Siebnetz (4) haftet.

8. Imprägnierter kosmetischer Gegenstand (2) gemäß einem der vorstehenden Ansprüche, wobei das Siebnetz (4) Löcher umfasst, die jeweils eine Oberfläche von 0,05 mm² bis 0,50 mm² aufweisen.

9. Imprägnierter kosmetischer Gegenstand (2) gemäß einem der vorstehenden Ansprüche mit einer Härte in dem Bereich von 13 bis 70, wie gemessen mit einem Durometer-Härteprüfer, Typ F.

10. Imprägnierter kosmetischer Gegenstand (2) gemäß einem der vorstehenden Ansprüche, wobei die kosmetische Zusammensetzung ein UV-Absorptionsmittel oder ein Konservierungsmittel umfasst.

11. Imprägnierter kosmetischer Gegenstand (2) gemäß einem der vorstehenden Ansprüche, wobei die Viskosität der kosmetischen Zusammensetzung zwischen 1.000 und 20.000 cps beträgt.

12. Verfahren zur Herstellung eines imprägnierten kosmetischen Gegenstands (2) gemäß einem der vorstehenden Ansprüche, umfassend die Schritte:
(i) Herstellen von Schichten von Faserträgern (3),
(ii) Schneiden der Schichten von Faserträgern (3),
(iii) Anordnen eines Siebnetzes (4) auf den Schichten von Faserträgern (3),
(iv) Anhaften des Siebnetzes (4) an die Faserträger (3),
(v) Imprägnieren einer kosmetischen Zusammensetzung.

13. Kosmetisches Produkt (10), umfassend:
- einen imprägnierten kosmetischen Gegenstand (2) gemäß einem der Ansprüche 1 bis 11,
- einen Behälter (5) für den Gegenstand (2).

14. Kosmetisches Produkt (10) gemäß Anspruch 13, ferner umfassend ein kosmetisches Werkzeug zum Aufnehmen der kosmetischen Zusammensetzung.

15. Kosmetisches Produkt gemäß Anspruch 13 oder 14, ferner umfassend eine Matte (7), die unter dem imprägnierten kosmetischen Gegenstand (2) angeordnet ist.

## Revendications

1. Article cosmétique imprégné (2) comprenant :
- un filet écran (4),
- un support fibreux (3) imprégné d'une composition cosmétique,
l'article ayant :
- une épaisseur de 1 mm à 200 mm,
- une densité surfacique de 30 g/m² à 5 500 g/m²,
- une élasticité de 50 % à 100 %,
et le filet écran (4) étant conçu pour former une surface de prélèvement de la composition cosmétique dont est imprégné le support fibreux (3) quand elle passe dans les trous du filet écran (4) .

2. Article cosmétique imprégné (2) selon la revendication 1, dans lequel le support fibreux (3) comprend un matériau non tissé.

3. Article cosmétique imprégné (2) selon l'une quelconque des revendications précédentes, dans lequel le support fibreux (3) est fabriqué avec des fibres de polyéthylène, des fibres de polypropylène, des fibres de téréphtalate de polyéthylène, ou un mélange de celles-ci.

4. Article cosmétique imprégné (2) selon l'une quelconque des revendications précédentes, dans lequel les fibres ont un diamètre de 1 µm à 100 µm.

5. Article cosmétique imprégné (2) selon l'une quelconque des revendications précédentes, dans lequel le support fibreux (3) comprend des cavités d'air et le pourcentage volumique des cavités d'air dans le support fibreux est de 35 % à 90 %.

6. Article cosmétique imprégné (2) selon l'une quelconque des revendications précédentes, dans lequel le filet écran (4) comprend une polyoléfine.

7. Article cosmétique imprégné (2) selon l'une quelconque des revendications précédentes, dans lequel le support fibreux (3) adhère au filet écran (4).

8. Article cosmétique imprégné (2) selon l'une quelconque des revendications précédentes, le filet écran (4) comprenant des trous ayant chacun une surface de 0,05 mm² à 0,50 mm².

9. Article cosmétique imprégné (2) selon l'une quelconque des revendications précédentes, ayant une dureté de 13 à 70, mesurée avec un appareil de mesure de dureté qui est un duromètre de type F.

10. Article cosmétique imprégné (2) selon l'une quelconque des revendications précédentes, dans lequel la composition cosmétique comprend un agent absorbeur d'UV ou un conservateur.

11. Article cosmétique imprégné (2) selon l'une quelconque des revendications précédentes, dans lequel la viscosité de la composition cosmétique est de 1 000 à 20 000 cps.

12. Procédé de fabrication d'un article cosmétique imprégné (2) selon l'une quelconque des revendications précédentes, comprenant les étapes qui consistent à :
(i) produire des couches de supports fibreux (3),
(ii) découper les couches de supports fibreux (3),
(iii) placer un filet écran (4) sur les couches de supports fibreux (3),
(iv) faire adhérer le filet écran (4) aux supports fibreux (3),
(v) imprégner d'une composition cosmétique.

13. Produit cosmétique (10) comprenant :
- un article cosmétique imprégné (2) selon l'une quelconque des revendications 1 à 11,
- un contenant (5) pour l'article (2).

14. Produit cosmétique (10) selon la revendication 13, comprenant en outre un outil cosmétique pour prélever la composition cosmétique.

15. Produit cosmétique selon la revendication 13 ou 14, comprenant en outre un tapis (7) positionné sous l'article cosmétique imprégné (2).
